# EUROPEAN PATENT SPECIFICATION

(11) **EP 2 178 841 B1**
(45) Date of publication and mention of the grant of the patent: **02.11.2011**
(21) Application number: 08773959.5
(22) Date of filing: 10.07.2008
(51) Int. Cl.: C07D 215/18, C07D 405/10

(54) **PROCESS AND INTERMEDIATE FOR THE PRODUCTION OF A TERTIARY ALCOHOL AS AN INTERMEDIATE IN THE SYNTHESIS OF MONTELUKAST**
VERFAHREN UND ZWISCHENPRODUKT ZUR HERSTELLUNG EINES TERTIÄREN ALKOHOLS ALS ZWISCHENPRODUKT IN DER SYNTHESE VON MONTELUKAST
PROCÉDÉ ET INTERMÉDIAIRE POUR LA PRÉPARATION D'UN INTERMÉDIAIRE DANS LA PRÉPARATION DE MONTELUKAST

(30) Priority: 13.07.2007 EP 07013810
(43) Date of publication of application: 28.04.2010
(73) Proprietor: Lonza Ltd, 4052 Basel (CH)
(72) Inventor: McGARRITY, John, CH-3902 Brig-Glis (CH); DJOJO, Francis, CH-3930 Visp (CH)
(86) International application number: PCT/EP2008/005637
(87) International publication number: WO 2009/010230

(56) References cited:
- WO-A-2007/057225
- US-A- 5 266 568
- DUFRESNE C ET AL: "Synthesis of montelukast (MK-0476) metabolic oxidation products" JOURNAL OF ORGANIC CHEMISTRY, AMERICAN CHEMICAL SOCIETY. EASTON, US, vol. 61, no. 24, 1996, pages 8518-8525, XP002284162 ISSN: 0022-3263
- ROBERT D. LARSON ET AL.: "Practical Route to a New Class of LTD4 Receptor Antagonists" JOURNAL OF ORGANIC CHEMISTRY, vol. 61, no. 10, 1996, pages 3398-3405, XP002462200
- CONLON D A ET AL: "INSIGHTS INTO THE CERIUM CHLORIDE-CATALYZED GRIGNARD ADDITION TO ESTERS" ADVANCED SYNTHESIS AND CATALYSIS, WILEY, WEINHEIM, DE, vol. 346, no. 11, 2004, pages 1307-1315, XP009058825 ISSN: 1615-4169
- EGEKEZE, JOHN O. ET AL: "Kinetic Analysis and Subambient Temperature Chromatography of an Active Ester" ANALYTICAL CHEMISTRY , 67(13), 2292-5 CODEN: ANCHAM; ISSN: 0003-2700, 1995, XP002462201
- GARY L. GRUNEWALD ET AL.: "A New Procedure for Regioselective Synthesis of 8,9-Dichloro-2,3,4,5-tetrahdro-1H-2-benzaz epine (LY134046) and its 3-Methyl Analogue as Inhibitors of Phenylethanolamine N-Methyltransferase (PNMT)" JOURNAL OF HETEROCYCLIC CHEMISTRY, vol. 28, 1991, pages 1587-1592, XP002514775

## Description

The invention relates to a process for the production of a tertiary alcohol of formula 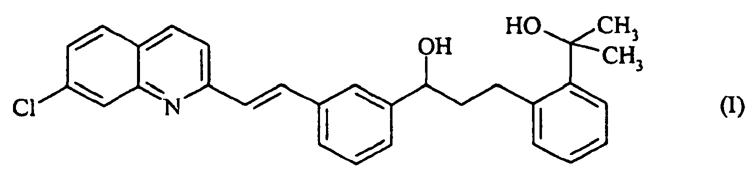 namely, α-[3-[(1*E*)-2-(7-chloro-2-quinolinyl)ethenyl]phenyl]-2-(1-hydroxy-1-methylethyl)benzenepropanol, the (α*S*)-stereoisomer of which is a key intermediate in the synthesis of the pharmaceutically active compound known as montelukast (1-[[[(1*R*)-1-[3-[(1*E*)-2-(7-chloro-2-quinolinyl)ethenyl]phenyl]-3-[2-(1-hydroxy-1-methylethyl)phenylpropyl]thio]methyl]cyclopropaneacetic acid). In further relates to a new intermediate of said process and a method for its preparation.

A known synthesis of (α*S*)-α-[3-[(1*E*)-2-(7-chloro-2-quinolinyl)ethenyl]phenyl]-2-(1-hydroxy-1-methylethyl)benzenepropanol is based on the reaction of a carboxylic ester with two equivalents of a Grignard reagent. However, the yields are not always satisfactory as undesired reactions compete with the formation of the alcohol and result in the formation of byproducts, in particular when an alkylmagnesium chloride is used as Grignard reagent (D. A. Conlon et al., Adv. Synth. Catal. 2004, 346, 1307-1315). It has been found that "nearly anhydrous" activated cerium trichloride has a beneficial effect on the above reaction, which has been postulated to be due to suppression of the enolization of the ketone intermediate. The water content and activation method of the cerium trichloride as well as its crystal habit have been found to be critical. Moreover, the activation of the cerium chloride is somewhat tedious and the activated cerium chloride is sparingly soluble in ethereal solvents such as tetrahydrofuran which results in a heterogeneous reaction mixture. In the preparation of the above montelukast intermediate the starting material (which is available as a monohydrate) has first to be carefully dried (e.g. by azeotropic distillation), but nevertheless, about 5 equivalents of methylmagnesium chloride are required, instead of the theoretical amount of 3 equivalents (WO 95/18107 A1).

WO 2007/057225 A2 discloses a method for the preparation of (α*R*)-α-[3-[(1*E*)-2-(7-chloro-2-quinolinyl)ethenyl]phenyl]-2-(1-hydroxy-1-methylethyl)benzenepropanethiol, the thio-analogue of I. The synthesis starts from methyl 2-[(3*S*)-3-[3-[(1*E*)-2-(7-chloro-2-quinolinyl)-ethenyl]phenyl]-3-hydroxypropyl]benzoate monohydrate (cf. formula IV below) which is first converted into the anhydrous form by azeotropic dehydration, then mesylated at its secondary alcohol moiety, followed by reaction with thioacetic acid to yield the corresponding thioacetate, which in turn is reacted with methylmagnesium halide to give an ε-thiolactone. The thiolactone is reacted with methylmagnesium chloride and cerium(III) chloride to give the desired tertiary alcohol having a secondary thiol group. The synthesis requires four reaction steps and an azeotropic dehydration and, in the last step, more than 3 moles of cerium (III) chloride per mole of thiolactone.

It is an object of the present invention to provide an improved method for the preparation of the tertiary alcohol α-[3-[(1*E*)-2-(7-chloro-2-quinolinyl)ethenyl]phenyl]-2-(1-hydroxy-1-methylethyl)benzenepropanol (I) from a corresponding carboxylic ester and a Grignard reagent which gives high yields of the desired product even if the chloride form of the Grignard reagent is used. The method should not involve tedious activation steps, large amounts of rare earth-metal compounds, heterogeneous reaction mixtures or cumbersome work-up procedures.
Applicants have found that the desired product is readily available by reacting the lactone of formula 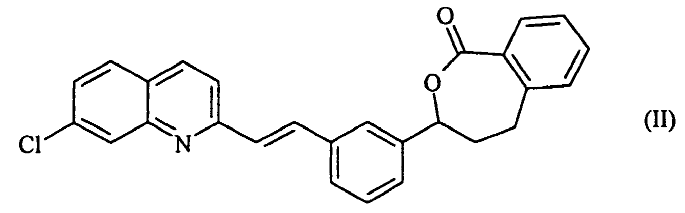 namely, 3-[3-[(*E*)-2-(7-chloro-2-quinolinyl)ethenyl]phenyl]-4,5-dihydro-3*H* benzo[*c*]oxepin-1-one,
with a Grignard reagent of formula

CH₃MgX (III),

wherein X is chlorine, bromine or iodine,
in an ethereal solvent in the presence of lanthanum trichloride and lithium chloride.

The term "ethereal solvent" is to be understood to mean any solvent or solvent mixture comprising a substantial amount of an acyclic or cyclic ether that is liquid at the reaction temperature, such as diethyl ether, dibutyl ether, methyl *tert*-butyl ether, dimethoxyethane, tetrahydrofuran (THF), 1,4-dioxane and the like. It also includes cyclic acetals such as 1,3-dioxolane or 1,3-dioxane.

The lithium chloride solubilizes the lanthanum trichloride, resulting in a true solution of the two salts in the ethereal solvent and thus in a homogeneous reaction mixture. In a preferred embodiment, lanthanum trichloride and lithium chloride are present in a molar ratio of 1:2 or less. A tetrahydrofuran solution of LaCl₃ and LiCl in a molar ratio of 1:2 is commercially available from Chemetall GmbH, Frankfurt (Main), Germany.

The halogen component X of the Grignard reagent III is preferably chlorine.

Most preferably, the secondary alcohol group and the carbon atom in position 3 of the oxepine ring, respectively, of the above structures I and II have *S*-configuration to make them suitable as intermediates in the synthesis of (R)-montelukast.

The ethereal solvent used in the process of the invention is preferably tetrahydrofuran alone or a mixture of tetrahydrofuran and an inert solvent such as an aliphatic or aromatic hydrocarbon.

The reaction temperature can be in the range that is commonly employed in Grignard reactions, it is preferably between -20 °C and room temperature, more preferably from -10 °C to +10 °C.

The work-up of the reaction mixture can be accomplished according to the methods commonly used in the art, e.g. by quenching with water or weak aqueous acids and extracting the product with a suitable solvent.

A particular advantage of the process according to the invention resides in the fact that, in contrast to the hydroxyester conventionally used as starting material (cf. WO 95/18107 A1), the lactone II has no active hydrogen that would result in the consumption of another equivalent of Grignard reagent. Another advantage resides in the fact that the amount of lanthanum chloride required is substantially lower than the amount of cerium chloride employed in the prior art process. The prior art process used cerium trichloride in a molar ratio of CeCl₃/hydroxyester starting material of about 1:1 while the process of the present invention can be carried out with substantially lower lanthanum trichloride/lactone molar ratios. This is especially advantageous in the work-up of the reaction mixture since the amount of magnesium and rare earth metal-containing wastes is quite substantially reduced.

In a preferred embodiment, the lanthanum trichloride/lactone molar ratio is between 1:2 and 1:10, more preferably between 1:3 and 1:5.

The lactone of formula II is a novel compound and likewise an object of the invention.

In a preferred embodiment, the carbon atom in position 3 of the oxepine ring of the lactone II has S-configuration.

Further objects of the invention are processes for the preparation of the lactone II. Applicants have surprisingly found that this compound is obtained in high selectivity when a carboxylic ester of formula 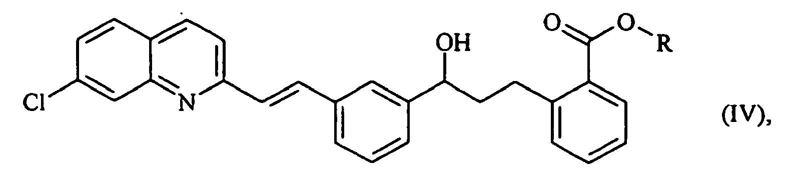 wherein R is C₁₋₁₀ alkyl, aryl or arylalkyl,
is reacted with a Grignard reagent of formula

R¹MgCl (V)

wherein R¹ is C₁₋₄ alkyl,
in an ethereal solvent in the absence of a lanthanoid compound, such as cerium or lanthanum chloride.

In a preferred embodiment, R¹ is methyl.

The term "C_{1-*n*} alkyl" is here to be understood to comprise any linear or branched alkyl group having from 1 to n carbon atoms. For example, the term "C₁₋₄ alkyl" comprises methyl, ethyl, propyl, isopropyl, butyl, isobutyl, *sec*-butyl and *tert*-butyl. In addition to the beforementioned, the term "C₁₋₁₀ alkyl" comprises groups such as pentyl, isopentyl, neopentyl, hexyl, isohexyl, heptyl, octyl, nonyl, decyl and the like.

The term "aryl" is to be understood to comprise any mono-, bi- or polycarbocyclic group comprising at least one aromatic ring, such as phenyl, naphthyl, anthracenyl, phenanthryl, biphenylyl, fluorenyl, tetrahydronaphthalenyl and the like. A preferred meaning of "aryl" is phenyl.

The term "arylalkyl" is to be understood to comprise an alkyl group, and in particular a C₁₋₄ alkyl group, which is substituted with one of the groups mentioned above under "aryl". The most preferred meaning of arylalkyl is benzyl.

In an alternative process which is also an object of the invention, the lactone II can be prepared by reacting the carboxylic ester IV with a strong base, such as a C₁₋₄-alkoxide of an alkali or alkaline earth metal. Preferred C₁₋₄-alkoxides are *tert-*butoxides, in particular sodium, potassium or magnesium *tert-*butoxides.

In a preferred embodiment, the group R in the ester moiety is methyl.

More preferably, the methyl ester is employed in the form of its monohydrate.
Both the carbon atom in position 3 of the oxepin ring in formula II and the secondary alcohol group in formula IV are preferably in the *S*-configuration.

The following non-limiting examples will illustrate the process of the invention.

### Example 1

### (3S)-3-[3-[(E)-2-(7-Chloro-2-quinolinyl)ethenyl]phenyl]-4,5-dihydro-3H-benzo[c]oxepin-1-one

A suspension of methyl 2-[(3*S*)-3-[3-[(1*E*)-2-(7-chloro-2-quinolinyl)ethenyl]phenyl]-3-hydroxypropyl]benzoate monohydrate (18.32 g, 38.5 mmol) in 60 mL THF and 210 mL toluene was cooled to -5 °C in a 250 mL double-jacketed reactor under nitrogen. Methylmagnesium chloride (3 M solution in THF, 53.4 mL, 160 mmol) was added dropwise during 25 min while maintaining the temperature at -5 °C. The brown suspension was stirred at 0 °C for 4 h and at 20 °C for 24 h. The reaction mixture was added during 15 min to 2 M aqueous acetic acid which had been pre-cooled to 5 °C. During the addition the temperature rose to 12 °C. The mixture was stirred at this temperature for another 5 min, and the phases were separated. The aqueous phase was discarded and the organic phase was washed with 10% aqueous sodium carbonate solution, and then with 1 % aqueous sodium carbonate solution (320 mL each). The solution was evaporated *in vacuo* (40 °C, 30 mbar) to yield 24 g residue which was dissolved in THF (15 mL) at 45 °C. Heptane (41 mL) was added dropwise to this solution. The suspension formed was cooled to 0 °C, filtered, washed with heptane (30 mL) and dried to yield 8.57 g beige solid.
m.p.: 160 °C
IR (KBr): = 2931, 1714, 1608, 1497, 1455, 1410, 1297, 1251, 1121, 1084, 1039, 927, 875, 832, 799, 774, 755, 731, 693 cm⁻¹
¹H NMR (DMSO-d₆, 500 MHz): δ = 2.28 (m, 1H), 2.45 (m, 1H), 2.98 (m, 1H), 3.04 (m, 1H), 5.17 (dd, *J* = 12.2, 4.9 Hz; 1H), 7.45 (m, 3H), 7.49 (m, 1H), 7.52 (d, *J* = 16.6 Hz, 1H), 7.58 (dd, *J* = 8.8, 1.9 Hz; 1H), 7.63 (t, *J* = 7.5 Hz, 1H), 7.68 (m, 1H), 7.71 (d, *J* = 7.3 Hz, 1H), 7,85 (s, 1H), 7.87 (d, *J* = 16.6 Hz, 1H), 7.88 (d, *J* = 8.8 Hz, 1H), 7.98 (d, *J* = 8.8 Hz, 1H), 8.0 (d, *J* = 1.5 Hz, 1H), 8.38 (d, *J* = 8.8 Hz, 1H).
¹³C NMR (DMSO-d₆, 126 MHz): δ = 29.24, 35.29, 78.86, 120.28, 125.20, 125.55, 126.61, 126.81, 127.16, 127.28, 128.71, 128.89, 128.97, 129.66, 129.80, 131.42, 132.66, 134.25, 134.48, 136.20, 136.49, 137.60, 139.81, 147.96, 156.61, 170.01.

### Example 2

### (αS)-α-[3-[(1E)-2-(7-Chloro-2-quinolinyl)ethenyl]phenyl]-2-(1-hydroxy-1-methylethyl)-benzenepropanol

A solution of (3*S*)-3-[3-[(*E*)-2-(7-chloro-2-quinolinyl)ethenyl]phenyl]-4,5-dihydro-3*H*-benzo[*c*]oxepin-1-one (3.05 g, 7.2 mmol) in THF (45 mL) was cooled to 5 °C in a 250 mL double-jacketed reactor under nitrogen. Methylmagnesium chloride (3 M solution in THF, 7.11 g, 21.0 mmol) was added. Lanthanum chloride/lithium chloride (molar ratio 1:2, 16% solution in THF, 3.23 g, 1.6 mmol) was added with stirring at 5 °C. The reaction mixture was heated to 20 °C and stirred at that temperature for another 1 h. Completion of the reaction was ensured by HPLC control. The solution was cooled to below 10 °C, and 2 M aqueous acetic acid (50 ml) was added during 5 min, followed by methyl *tert*-butyl ether (50 mL). The phases were separated and the organic phase was washed first with 10% aqueous sodium carbonate solution (50 mL) and then with saturated brine (50 mL). The organic phase was concentrated *in vacuo* (40 °C, 280 mbar) to a weight of 6.83 g. Heptane (17 mL) was added dropwise to this residue during 1 h at 25 °C, then the suspension was cooled to 0 °C and stirred for 1 h. The precipitate was filtered, washed with heptane (10 mL) and dried *in vacuo* at 30 °C to yield 2.31 g light beige powder, purity (HPLC) 94.9%.
¹H NMR (DMSO-d₆, 500 MHz): δ = 1.51 (s, 3H); 1.52 (s, 3H); 2.00 (m, 2H), 2.96 (m, 1H); 3.10 (m, 1H); 4.72 (m, 1H); 4.94 (s, 1H); 5.36 (d, *J =* 4.4 Hz, 1H); 7.09 (t, *J* = 7.6 Hz, 1H); 7.14 (t, *J* = 7.8 Hz, 1H); 7.18 (d, *J* = 6.4 Hz, 1H); 7.41 (m, 2H); 7.44 (d, *J* = 7.9 Hz, 1H); 7.49 *(d, J =* 16.6 Hz, 1H); 7.56 (dd, *J* = *8.3,* 2.2 Hz, 1H); 7.62 (d, *J = 6.8* Hz, 1H); 7.77 (bs, 1H); 7.91 (d, *J* = 16.6 Hz, 1H); 7.92 (d, *J* = 8.7 Hz, 1H); 7.99 (d, *J* = 8.8 Hz, 1H); 8.03 (d, *J* = 2.0 Hz, 1H); 8.3 8 (d, *J* = 8.4 Hz, 1H).
¹³C NMR (DMSO-d₆, 126 MHz): δ = 29.82, 31.55, 31.57, 42.34, 71.60, 72.31, 120.24, 124.73, 124.88, 125.24, 125.51, 125.71, 126.22, 126.54, 127.16, 128.04, 128.49, 129.65, 130.82, 134.23, 135.20, 135.67, 136.43, 140.25, 146.66, 146.93, 147.99, 156.78.

### Example 3

### (3S)-3-[3-[(E)-2-(7-Chloro-2-quinolinyl)ethenyl]phenyl]-4,5-dihydro-3H-benzo[c]oxepin-1-one

In a 250 mL flask, methyl 2-[(3*S*)-3-[3-[(1*E*)-2-(7-chloro-2-quinolinyl)ethenyl]phenyl]-3-hydroxypropyl]benzoate monohydrate (9.4 g, 20 mmol) was suspended in toluene (100 mL) and heated to reflux. 52 mL of solvent was distilled from the solution at 110 °C and normal pressure. Karl Fischer analysis showed that the water content of the solution was 0.023% (1 mL of solution was drawn). 4 Å molecular sieve (3 g) was added to the solution. Then magnesium *tert-but*oxide (7.5 g, 40 mmol) was added. The reaction mixture was stirred at about 20 °C while the reaction was monitored by HPLC. After the reaction was completed, THF (20 mL) and water (5 mL) was added to quench the reaction. The solid was filtered and washed with THF (30 mL). The filtrate was concentrated to 30 mL. n-Heptane (20 mL) was added dropwise while stirring and then the suspension formed was cooled to 0 °C. The solid product was filtered off and dried at 25 °C under vacuum.
Yield: 5.2 g (61.8%), purity (HPLC) 98.8%.

## Claims

1. A process for the production of α-[3-[(1*E*)-2-(7-chloro-2-quinolinyl)ethenyl]phenyl]-2-(1-hydroxy-1-methylethyl)benzenepropanol of formula 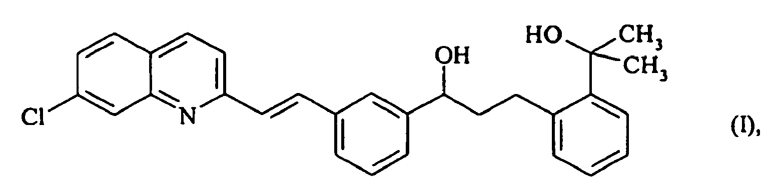 by reacting the lactone 3-[3-[(*E*)-2-(7-chloro-2-quinolinyl)ethenyl]phenyl]-4,5-dihydro-3*H*-benzo[*c*]oxepin-1-one of formula 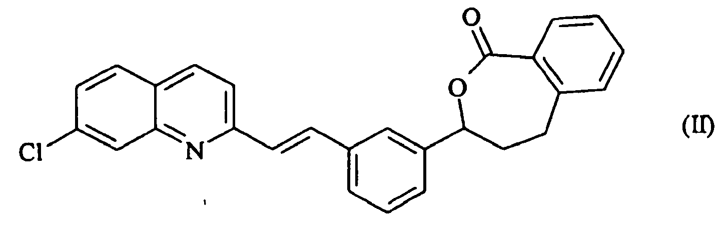 with a Grignard reagent of formula
CH₃MgX (III),
wherein X is chlorine, bromine or iodine,
in an ethereal solvent in the presence of lanthanum trichloride and lithium chloride.

2. The process of claim 1, wherein lanthanum trichloride and lithium chloride are present in a molar ratio of 1:2.

3. The process of claim 1 or 2, wherein the secondary alcohol group in I and the carbon atom in position 3 of the oxepine ring have S-configuration.

4. The process of any of claims 1 to 3, wherein the molar ratio of lanthanum trichloride to lactone (II) is from 1:2 to 1:10.

5. The process of claim 4, wherein the molar ratio of lanthanum trichloride to lactone (II) is from 1:3 to 1:5.

6. 3-[3-[(*E*)-2-(7-Chloro-2-quinolinyl)ethenyl]phenyl]-4,5-dihydro-3*H* benzo[*c*]oxepin-1-one of formula

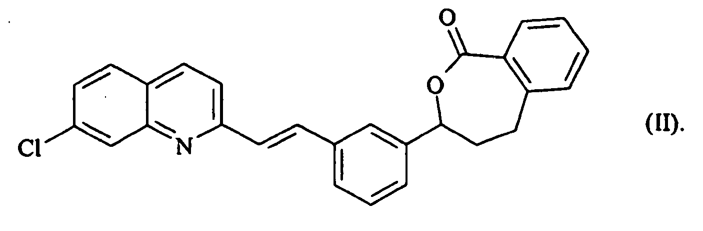

7. The compound of claim 6, which is (3*S*)-3-[3-[(*E*)-2-(7-chloro-2-quinolinyl)ethenyl]-phenyl]-4,5-dihydro-3*H*.benzo[*c*]oxepin-1-one of formula

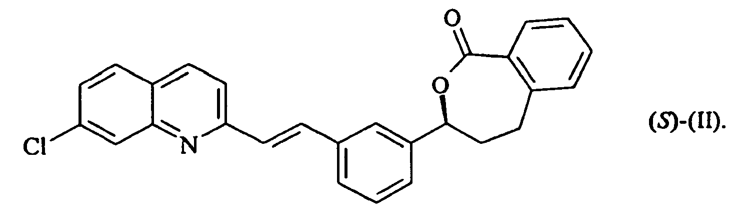

8. A process for the preparation of 3-[3-[(*E*)-2-(7-chloro-2-quinolinyl)ethenyl]phenyl]-4,5-dihydro-3*H-*benzo[*c*]oxepin-1-one of formula 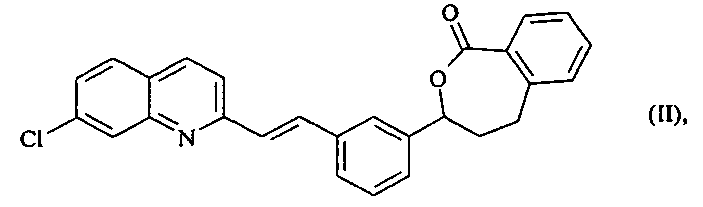 comprising reacting a carboxylic ester of formula 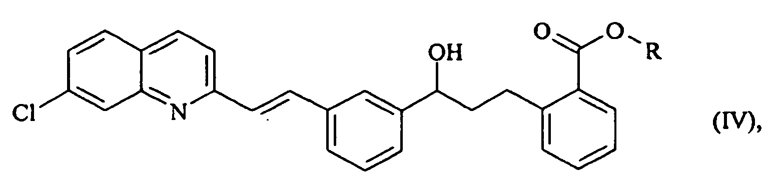 wherein R is C₁₋₁₀ alkyl, aryl or arylalkyl,
with a Grignard reagent of formula
R¹MgCl (V),
wherein R¹ is C₁₋₄ alkyl,
in an ethereal solvent in the absence of a lanthanoid compound.

9. The process of claim 8, wherein R¹ is methyl.

10. A process for the preparation of 3-[3-[(*E*)-2-(7-chloro-2-quinolinyl)ethenyl]phenyl]-4,5-dihydro-3*H*-benzo[*c*]oxepin-1-one of formula 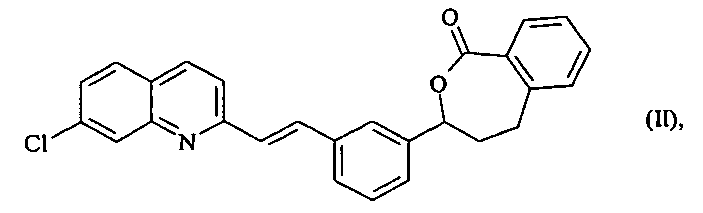 comprising reacting a carboxylic ester of formula 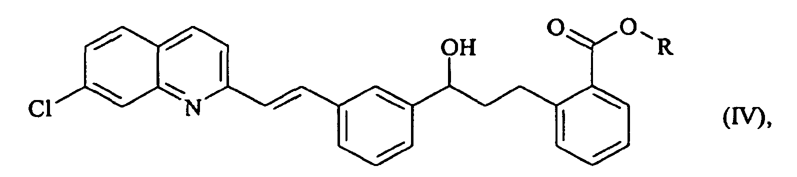 wherein R is C₁₋₁₀ alkyl, aryl or arylalkyl,
with a strong base.

11. The process of claim 10, wherein the strong base is a C₁₋₄-alkoxide of an alkali or alkaline earth metal.

12. The process of any of claims 8 to 11, wherein R is methyl.

13. The process of claim 12, wherein the carboxylic ester IV is in the monohydrate form.

14. The process of any of claims 8 to 13, wherein the carbon atom in position 3 of the oxepin ring in formula II and the secondary alcohol group in formula IV have S-configuration.

15. The process of any of claims 1 to 3 or 8 to 14, wherein the ethereal solvent is tetrahydrofuran or a mixture of tetrahydrofuran and an inert solvent.

## Patentansprüche

1. Verfahren zur Herstellung von α-[3-[(1*E*)-2-(7-Chlor-2-chinolinyl)ethenyl]phenyl]-2-(1-hydroxy-1-methylethyl)benzenpropanol der Formel 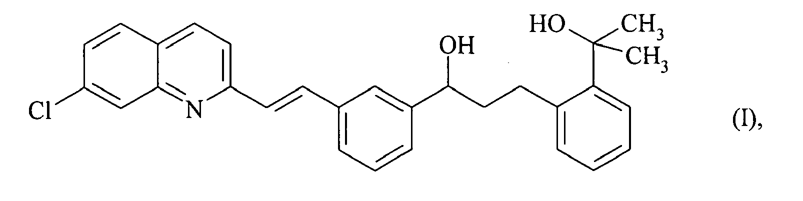 durch Umsetzung des Lactons 3-[3-[(*E*)-2-(7-Chloro-2-chinolinyl)ethenyl]phenyl]-4,5-dihydro-3*H-*benzo[*c*]oxepin-1-on der Formel 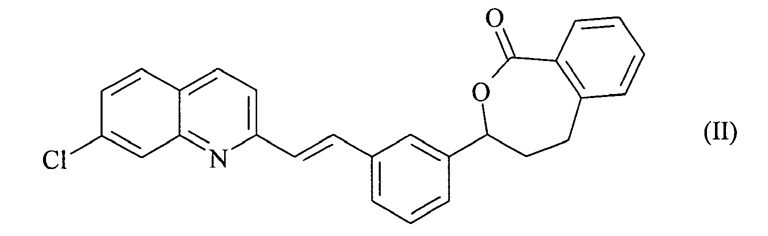 mit einem Grignard-Reagenz der Formel
CH₃MgX (III),
worin X Chlor, Brom oder Iod ist,
in einem etherischen Lösungsmittel in Gegenwart von Lanthantrichlorid und Lithiumchlorid.

2. Verfahren nach Anspruch 1, worin Lanthantrichlorid und Lithiumchlorid in einem molaren Verhältnis von 1:2 anwesend sind.

3. Verfahren nach Anspruch 1 oder 2, worin die sekundäre Alkoholgruppe in I und das Kohlenstoffatom in Position 3 des Oxepinrings die S-Konfiguration besitzen.

4. Verfahren nach einem der Ansprüche 1 bis 3, worin das molare Verhältnis von Lanthantrichlorid zu Lacton (II) von 1:2 bis 1:10 beträgt.

5. Verfahren nach Anspruch 4, worin das molare Verhältnis von Lanthantrichlorid zu Lacton (II) von 1:3 bis 1:5 beträgt.

6. 3-[3-[(*E*)-2-(7-Chlor-2-chinolinyl)ethenyl]phenyl]-4,5-dihydro-3*H*-benzo[*c*]oxepin-1-on der Formel

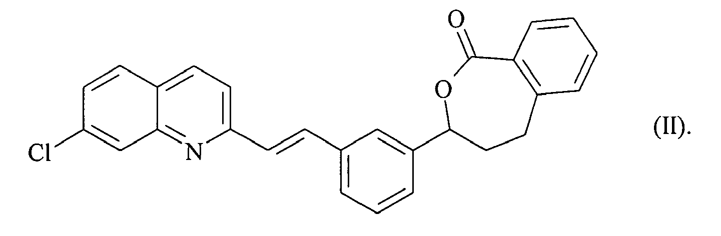

7. Verbindung nach Anspruch 6, welche (3*S*)-3-[3-[(*E*)-2-(7-Chlor-2-chinolinyl)ethenyl]-phenyl]-4,5-dihydro-3*H*-benzo[*c*]oxepin-1-on der Formel 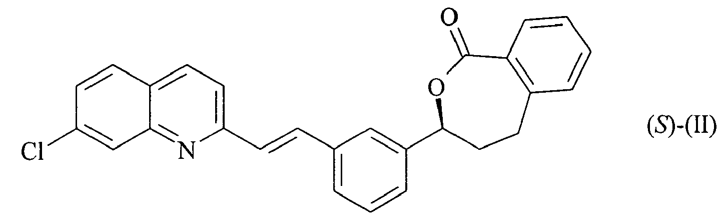 ist.

8. Verfahren zur Herstellung von 3-[3-[(*E*)-2-(7-Chlor-2-chinolinyl)ethenyl]phenyl]-4,5-dihydro-3*H-*benzo[*c*]oxepin-1-on der Formel 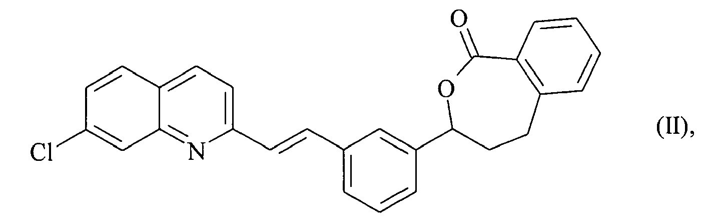 umfassend die Reaktion eines Carbonsäureesters der Formel 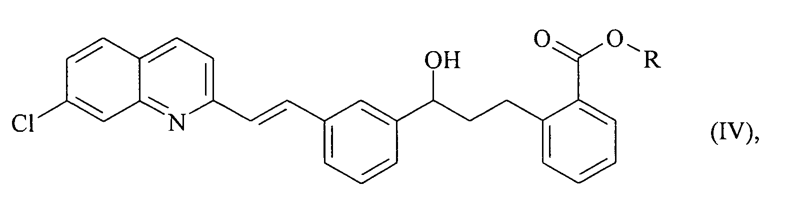 worin R C₁₋₁₀-Alkyl, Aryl oder Arylalkyl ist,
mit einem Grignard-Reagenz der Formel
R¹MgCl (V),
worin R¹ C₁₋₄-Alkyl ist,
in einem etherischen Lösungsmittel in Abwesenheit einer Lanthanoid-Verbindung.

9. Verfahren nach Anspruch 8, worin R^{l} Methyl ist.

10. Verfahren zur Herstellung von 3-[3-[(*E*)-2-(7-Chlor-2-chinolinyl)ethenyl]phenyl]-4,5-dihydro-3*H-*benzo[*c*]oxepin-1-on der Formel 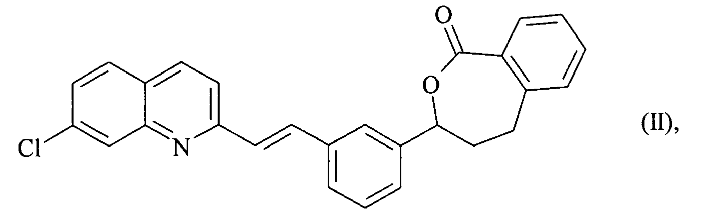 umfassend die Reaktion eines Carbonsäureesters der Formel 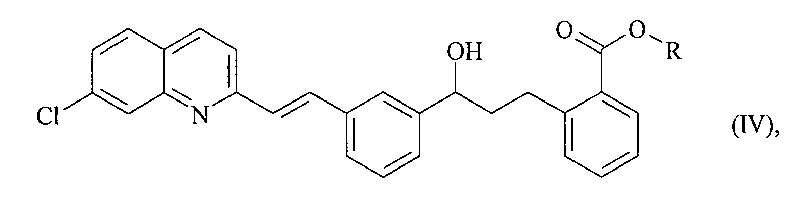 worin R C₁₋₁₀-Alkyl, Aryl oder Arylalkyl ist,
mit einer starken Base.

11. Verfahrem nach Anspruch 10, worin die starke Base ein C₁₋₄-Alkoxid eines Alkali- oder Erdalkalimetalls ist.

12. Verfahren nach einem der Ansprüche 8 bis 11, worin R Methyl ist.

13. Verfahren nach Anspruch 12, worin der Carbonsäureester IV in der Monohydratform vorliegt.

14. Verfahren nach einem der Ansprüche 8 bis 13, worin das Kohlenstoffatom in Position 3 des Oxepinrings in Formel II und die sekundäre Alkoholgruppe in Forml IV die *S*-Konfiguration besitzen.

15. Verfahren nach einem der Ansprüche 1 bis 3 oder 8 bis 14, worin das etherische Lösungsmittel Tetrahydrofuran oder ein Gemisch aus Tetrahydrofuran und einem inerten Lösungsmittel ist.

## Revendications

1. Procédé de fabrication d'α-[3-[(1*E*)-2-(7-chloro-2-quinolinyl)éthényl]phényl]-2-(1-hydroxy-1-méthyléthyl)benzènepropanol de formule 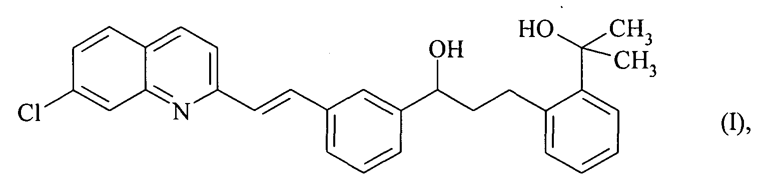 par mise en réaction de la lactone 3-[3-[(*E*)-2-(7-chloro-2-quinolinyl)éthényl]-phényl]-4,5-dihydro-3*H*-benzo[*c*]oxépin-1-one de formule 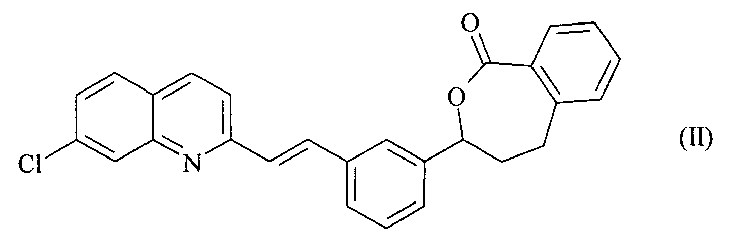 avec un réactif de Grignard de formule
CH₃MgX (III),
dans laquelle X est le chlore, le brome ou l'iode,
dans un solvant éthéré en la présence de trichlorure de lanthane et de chlorure de lithium.

2. Procédé selon la revendication 1, dans lequel le trichlorure de lanthane et de chlorure de lithium sont présents en un rapport molaire de 1:2.

3. Procédé selon la revendication 1 ou 2, dans lequel le groupe alcool secondaire dans I et l'atome de carbone en position 3 du cycle oxépine ont la configuration S.

4. Procédé selon l'une quelconque des revendications 1 à 3, dans lequel le rapport molaire entre le trichlorure de lanthane et la lactone (II) est de 1:2 à 1:10.

5. Procédé selon la revendication 4, dans lequel le rapport molaire entre le trichlorure de lanthane et la lactone (II) est de 1:3 à 1:5.

6. 3-[3-[(*E*)-2-(7-Chloro-2-quinolinyl)éthényl]phényl]-4,5-dihydro-3*H*-benzo[*c*]oxépin-1-one de formule

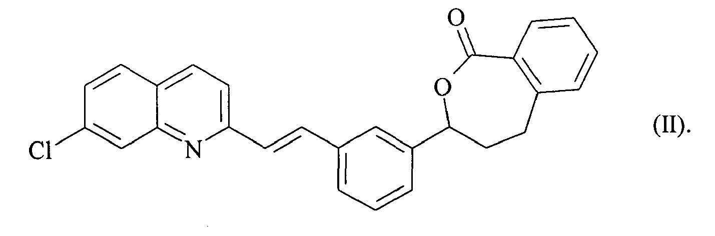

7. Composé selon la revendication 6, qui est la (3*S*)-3-[3-[(*E*)-2-(7-chloro-2-quinolinyl)éthényl]phényl]-4,5-dihydro-3*H*-benzo[*c*]oxépin-1-one de formule

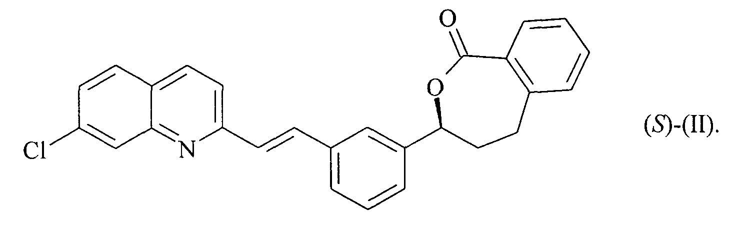

8. Procédé de préparation de 3-[3-[(*E*)-2-(7-chloro-2-quinolinyl)éthényl]phényl]-4,5-dihydro-3*H*-benzo[*c*]oxépin-1-one de formule 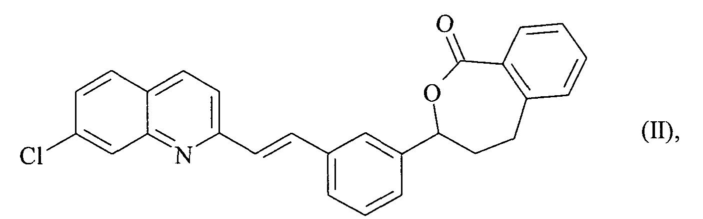 comprenant la mise en réaction d'un ester carboxylique de formule 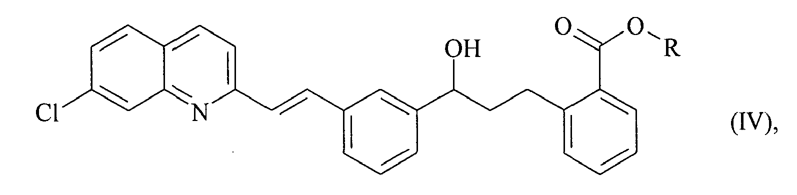 dans laquelle R est un alkyle en C₁₋₁₀, un aryle ou un arylalkyle,
avec un réactif de Grignard de formule
R¹MgCl (V),
dans laquelle R¹ est un alkyle en C₁₋₄,
dans un solvant éthéré en l'absence d'un composé de lanthanide.

9. Procédé selon la revendication 8, dans lequel R¹ est le méthyle.

10. Procédé de préparation de 3-[3-[(*E*)-2-(7-chloro-2-quinolinyl)éthényl]phényl]-4,5-dihydro-3*H*-benzo[*c*]oxépin-1-one de formule 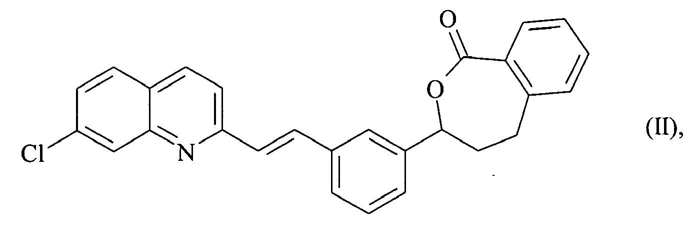 comprenant la mise en réaction d'un ester carboxylique de formule 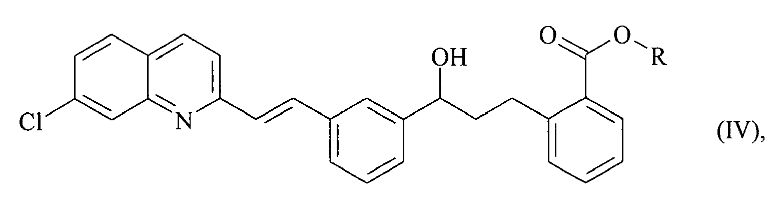 dans laquelle R est un alkyle en C₁₋₁₀, un aryle ou un arylalkyle,
avec une base forte.

11. Procédé selon la revendication 10, dans lequel la base forte est un alcoxyde en C₁₋₄ d'un métal alcalin ou alcalino-terreux.

12. Procédé selon l'une quelconque des revendications 8 à 11, dans lequel R est le méthyle.

13. Procédé selon la revendication 12, dans lequel l'ester carboxylique IV est sous la forme monohydrate.

14. Procédé selon l'une quelconque des revendications 8 à 13, dans lequel l'atome de carbone en position 3 du cycle oxépine dans la formule II et le groupe alcool secondaire dans la formule IV ont la configuration S.

15. Procédé selon l'une quelconque des revendications 1 à 3 ou 8 à 14, dans lequel le solvant éthéré est le tétrahydrofurane ou un mélange de tétrahydrofurane et d'un solvant inerte.
